# EUROPEAN PATENT SPECIFICATION

(11) **EP 3 936 102 B1**
(45) Date of publication and mention of the grant of the patent: **08.10.2025**
(21) Application number: 20184417.2
(22) Date of filing: 07.07.2020
(51) Int. Cl.: A61F 13/42

(54) **MONITORING DEVICE ATTACHABLE TO AN ABSORBENT ARTICLE, AND RESPECTIVE METHOD AND ABSORBENT ARTICLE**
ÜBERWACHUNGSVORRICHTUNG, DIE AN EINEM ABSORBIERENDEN ARTIKEL ANBRINGBAR IST, UND ENTSPRECHENDES VERFAHREN UND ABSORBIERENDER ARTIKEL
DISPOSITIF DE SURVEILLANCE POUVANT ÊTRE FIXÉ SUR UN ARTICLE ABSORBANT, PROCÉDÉ CORRESPONDANT ET ARTICLE ABSORBANT

(43) Date of publication of application: 12.01.2022
(73) Proprietor: Ontex BV, 9255 Buggenhout (BE); Ontex Group NV, 9320 Erembodegem (BE)
(72) Inventor: HELLMOLD, Jens, 59269 Beckum (DE); HEIRMAN, Lisa, 9250 Waasmunster (BE); MARRES, Bert, 9300 Aalst (BE)
(74) Representative: Saurat, Thibault

(56) References cited:
- WO-A1-2015/094065
- WO-A1-2017/143396
- WO-A1-2019/032991
- WO-A2-2013/023054

## Description

The invention relates to a monitoring device attachable to an absorbent article comprising a conductive pattern, a method for using such a monitoring device, and an absorbent article comprising said conductive pattern. Generally, in times of an increasing number of elderly and care-dependent people, there is a growing need of a monitoring device attachable to an absorbent article comprising a conductive pattern, a method for using such a monitoring device, and an absorbent article comprising said conductive pattern in order to handle incontinence in a particularly efficient manner.

EP 2 979 669 A1 relates to a system for monitoring incontinence in one or more subjects. The system comprises display means input means operable by a user and one or more transmitters, each transmitter being associated with one or more subjects being monitored. The one or more transmitters is configured to transmit signals containing continence-related data for the one or more subjects, wherein the continence-related data has been obtained over time from a continence sensor associated with an absorbent article worn by each respective subject. A receiver unit is configured to receive signals from the one or more transmitters. Processing means are in communication with the receiver unit. The processing means include a display processor configured to process the received signals and communicate display information to the display means for display of a visual representation of continence-related information derived from continence sensors in the absorbent articles worn by the one or more subjects being monitored. Disadvantageously, especially due to the lack of a verification of proper attachment of the transmitters and/or continence sensors with respect to the corresponding absorbent article, measurement errors can occur, which leads to inaccuracies and inefficiencies. WO2019032991 discloses a further device for monitoring incontinence.

Accordingly, there is an object to provide a monitoring device attachable to an absorbent article comprising a conductive pattern, a method for using such a monitoring device, and an absorbent article comprising a conductive pattern, whereby both a particularly high efficiency and a notably high accuracy are ensured.

This object is solved by the features of claim 1 for a monitoring device attachable to an absorbent article comprising a conductive pattern, the features of claim 11 for a method for using such a monitoring device, and the features of claim 14 for an absorbent article comprising a conductive pattern.

According to a first aspect of the invention, a monitoring device attachable to an absorbent article comprising a conductive pattern is provided. Said monitoring device comprises at least three terminals contactable to the conductive pattern, wherein the terminals (11a, 11b, 11c, 11d, 11e, 11f) are arranged on the monitoring device (10) according to a predefined terminal pattern, and wherein the predefined terminal pattern comprises or is a matrix; and a processing unit connected to the at least three terminals. In this context, in the case that the monitoring device is attached to the absorbent article and the at least three terminals contact the conductive pattern at least partly, on the basis of the contact between at least a part of the at least three terminals and the conductive pattern, the processing unit is configured to acquire information for verifying a proper attachment of the monitoring device to the absorbent article and/or for identifying a type of the absorbent article and/or for determining a wetness and/or saturation status of the absorbent article. The acquired information comprises at least one measurement value of at least one of resistance, capacitance, inductance, impedance, or any combination thereof. Advantageously, measurements can be performed in a particularly high efficient and accurate manner with reduced complexity. It is noted that it might be particularly advantageous if the monitoring device comprises at least three terminals. Further advantageously, especially having all terminals in one plane ensures that they can effectively be in contact to a substrate in a simultaneous manner.

According to a first preferred implementation form of the first aspect of the invention, the monitoring device comprises at least four terminals, preferably six terminals, more preferably eight terminals, most preferably fourteen terminals. Advantageously, for instance, accuracy can further be increased.

According to a further preferred implementation form of the first aspect of the invention, the distance between the terminals with regard to each other is predefined. Advantageously, for instance, simplicity can further be increased, thereby reducing inefficiencies.

In this context, it is noted that it might be particularly advantageous if the matrix comprises or is a particular arrangement of elements in rows and/or columns. Additionally or alternatively, said elements may be not necessarily arranged in right angles. Further additionally or further alternatively, not necessarily all spots of the matrix may be occupied.

According to a further preferred implementation form of the first aspect of the invention, the matrix comprises two rows and/or two columns. Advantageously, for example, the respective product design can further be simplified, which saves costs. Further advantageously, especially by using two rows, the highest efficiency for using terminals is achieved because two terminals can be placed on one line (forming one column, each one within one row) that is aligned to the application direction of the monitoring device with respect to the absorbent article.

With respect to the rows, it is noted that a row may especially comprise or be a horizontal arrangement of elements, wherein said arrangement is not necessarily made onto a straight line. Additionally or a alternatively, a column may especially comprise or be an arrangement of elements in a direction that is substantially perpendicular to the direction of the rows.

According to a further preferred implementation form of the first aspect of the invention, for verifying the proper attachment of the monitoring device to the absorbent article, the acquired information comprises the result of a comparison between the at least one measurement value and at least one respective verification reference value, preferably at least one respective verification threshold value. Advantageously, for example, said value may preferably built-in to the respective firmware of the monitoring device.

According to a further preferred implementation form of the first aspect of the invention, for identifying the type of the absorbent article, the acquired information comprises the result of a comparison between the at least one measurement value and at least one respective identification reference value, preferably at least one respective identification threshold value, and/or the result of a type interpretation of the at least one measurement value. Advantageously, for instance, said interpretation may especially allow for highly flexible and accurate measurements.

According to a further preferred implementation form of the first aspect of the invention, for determining the wetness and/or saturation status of the absorbent article, the acquired information comprises the result of a comparison between the at least one measurement value and at least one respective determination reference value, preferably at least one respective determination threshold value, and/or the result of a status interpretation of the at least one measurement value. Advantageously, for example, said value may preferably built-in to the respective firmware of the monitoring device.

According to a further preferred implementation form of the first aspect of the invention, for verifying the proper attachment of the monitoring device to the absorbent article, the processing unit is configured to select at least two, preferably at least two pairs, of the terminals being relatively widely spaced or having the greatest distance with regard to each other for acquiring the information. Advantageously, for instance, the proper attachment can be verified in a highly accurate and efficient manner.

According to a further preferred implementation form of the first aspect of the invention, for verifying the proper attachment of the monitoring device to the absorbent article and/or for identifying the type of the absorbent article, the processing unit is configured to select at least two, preferably at least two pairs, of the terminals for acquiring the information in such a manner that the selected terminals are unambiguously shorted or left open by contacting the conductive pattern. Advantageously, for example, the proper attachment can unequivocally be verified. Further advantageously, the terminals may especially be arranged relatively to the conductive patterns so that only when the monitoring device is brought in targeted position, a conductive connection is made only where intended.

According to a further preferred implementation form of the first aspect of the invention, the monitoring device comprises a feedback unit. In this context, the feedback unit is configured to acoustically and/or optically and/or haptically notify a status with respect to at least one of the proper attachment of the monitoring device to the absorbent article, the type of the absorbent article, the wetness and/or saturation of the absorbent article, or any combination thereof to a user. Advantageously, for instance, the user is informed in a simple and efficient manner.

According to a second aspect of the invention, a method for using a monitoring device according to the first aspect of the invention or its preferred implementation forms is provided. Said method comprises the steps of attaching the monitoring device to an absorbent article comprising a conductive pattern, contacting the at least three terminals of the monitoring device to the conductive pattern, and verifying a proper attachment of the monitoring device to the absorbent article with the aid of the monitoring device. Advantageously, measurements can be performed in a particularly high efficient and accurate manner.

According to a first preferred implementation form of the second aspect of the invention, the method comprises the steps of repeating or correcting the attachment of the monitoring device to the absorbent article in the case of an improper attachment until the monitoring device is properly attached to the absorbent article. In addition to this or as an alternative, the method comprises the step of identifying the type of the absorbent article in the case of a proper attachment with the aid of the monitoring device. Advantageously, for instance, an improper attachment can be corrected in a highly accurate and efficient manner. Further advantageously, in the case of identifying the type of the absorbent article only after having verified proper attachment, a correct read-out of the product type identifier is ensured.

According to a second preferred implementation form of the second aspect of the invention, the method comprises the step of determining the wetness and/or saturation status of the absorbent article with the aid of the monitoring device especially on the basis of the type of the absorbent article and/or especially only after having verified the proper attachment of the monitoring device to the absorbent article. Advantageously, for example, measurements can be performed in a highly accurate and efficient manner. Further advantageously, for instance, a correct interpretation of the wetness and/or saturation status is ensured.

According to a third aspect of the invention, an absorbent article is provided. Said absorbent article comprises a conductive pattern contactable by at least three terminals of a monitoring device such as the monitoring device according to the first aspect of the invention. In this context, the conductive pattern comprises conductive tracks. Additionally, the conductive tracks are formed in such a manner that the conductive tracks interconnect the at least three terminals according to a predefined pattern if the conductive pattern is contacted by the at least two terminals of the monitoring device.

Advantageously, at least a part of the conductive tracks may preferably be arranged in the form of interlaced fingers or any kind thereof. It is noted that said part of the conductive tracks may especially be used for exudate monitoring.

In addition to this or as an alternative, at least a part of the conductive tracks may preferably be arranged in the form of a capacitor, especially a parallel-plate capacitor, or any kind thereof. Said part of the conductive tracks may preferably be used for exudate monitoring.

Further advantageously, the maximum distance between two, especially two parallel, conductive tracks is equal to the maximum distance between two terminals, preferably two pairs of the terminals. It is noted that said maximum distance may preferably be the width of the conductive pattern or the width of the monitoring device.

As a further advantage, at least one of the conductive tracks stops on a first predefined axis, preferably a first axis being parallel to the above-mentioned maximum distance. It is noted that especially in the context of the above-mentioned width, the first predefined axis may preferably be a certain height, especially a first height. Advantageously, it can be controlled if the respective terminals are not positioned too high against the corresponding conductive tracks.

In this context, it might be particularly advantageous if at least one of the conductive tracks stops on a second predefined axis, preferably a second axis being parallel to the above-mentioned maximum distance and not being equal to the first axis. It is noted that especially in the context of the above-mentioned first height, the second predefined axis may preferably be a second height being especially below the first height. Advantageously, it can be controlled if the respective terminals are not positioned too low against the corresponding conductive tracks.

According to a first preferred implementation form of the third aspect of the invention, the conductive pattern comprises an ink, preferably a carbon-based ink and/or a conductive polymer-based ink.

According to a second preferred implementation form of the third aspect of the invention, the ink comprises at least one of graphene, graphite, nano-carbon-tubes, polyacetylene, polypyrrole, polyaniline and copolymers thereof, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), poly(3,4-ethylenedioxythiophene) polystyrene sulfonate (PEDOT:PSS), or any combination thereof.

Exemplary embodiments of the invention are now further explained with respect to the drawings by way of example only, and not for limitation. In the drawings:
- Fig. 1: shows an exemplary embodiment of the inventive monitoring device;
- Fig. 2: shows a flow chart of a first exemplary embodiment of the inventive usage method;
- Fig. 3: shows the first exemplary embodiment of the inventive usage method according to Fig. 2 in greater detail;
- Fig. 4: shows an exemplary threshold for attachment verification in the context of a presence type;
- Fig. 5: shows an exemplary threshold for attachment verification in the context of an absence type;
- Fig. 6: shows an exemplary arrangement of terminals with attachment verification tracks at the extreme sides;
- Fig. 7: shows an exemplary arrangement of terminals with attachment verification tracks that stop at a certain height;
- Fig. 8: shows an exemplary arrangement of terminals with a track that stops at a second height below a first height;
- Fig. 9: shows an exemplary threshold for product identification;
- Fig. 10: shows exemplary value types for product identification;
- Fig. 11: shows an exemplary threshold for exudate monitoring;
- Fig. 12: shows exemplary value types for exudate monitoring;
- Fig. 13: shows an explanatory example case with eight terminals;
- Fig. 14: shows a non-exhaustive list of possible product identifiers for the explanatory example of Fig. 13;
- Fig. 15: shows an exemplary product design of the inventive monitoring device with 14 terminals and corresponding design of a conductive pattern;
- Fig. 16: shows the exemplary product design and the design of the conductive pattern according to Fig. 15 in the context of a track that stops at a second height below a first height;
- Fig. 17: shows three exemplary embodiments of product identification tracks;
- Fig. 18: shows an explanatory example case with six terminals;
- Fig. 19: shows a set of four possible product identifiers for the explanatory example of Fig. 18; and
- Fig. 20: shows a flow chart of a second exemplary embodiment of the inventive usage method.

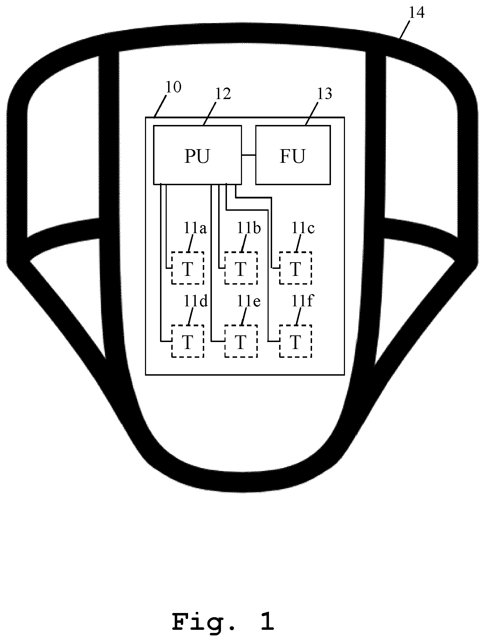

Firstly, Fig. 1 illustrates an exemplary embodiment of the inventive monitoring device 10 attachable to an absorbent article, exemplarily a diaper 14, comprising a conductive pattern. According to Fig. 1, said monitoring device 10 comprises at least two terminals, exemplarily six terminals 11a, 11b, 11c, 11d, 11e, 11f, contactable to the conductive pattern, and a processing unit 12 connected to the at least two terminals, exemplarily the six terminals 11a, 11b, 11c, 11d, 11e, 11f.

In this context, in the exemplary case that the monitoring device 10 is attached to the diaper 14 and the six terminals 11a, 11b, 11c, 11d, 11e, 11f contact the conductive pattern at least partly, on the basis of the contact between at least a part of the six terminals 11a, 11b, 11c, 11d, 11e, 11f and the conductive pattern, the processing unit 12 is configured to acquire information for verifying a proper attachment of the monitoring device 10 to the diaper 14 and/or for identifying a type of the diaper 14 and/or for determining a wetness and/or saturation status of the diaper 14.

Generally, it is noted that it might be particularly advantageous if the monitoring device 10 comprises at least four terminals, preferably six terminals as exemplarily shown in Fig. 1, more preferably eight terminals, most preferably fourteen terminals. As it can further be seen, the six terminals 11a, 11b, 11c, 11d, 11e, 11f are arranged on the monitoring device 10 according to a predefined terminal pattern. In addition to this, the six terminals 11a, 11b, 11c, 11d, 11e, 11f are arranged in the same plane.

Moreover, the predefined terminal pattern exemplarily is a matrix. In addition to this, the distance between the terminals 11a, 11b, 11c, 11d, 11e, 11f with regard to each other is predefined. In further addition to this, the matrix exemplarily comprises two lines and three columns.

Furthermore, it might be particularly advantageous if the absorbent article or the diaper 14, respectively, is equipped with a pocket area that comprises at least a part of the conductive pattern to which the terminals of the monitoring device 10 are to be connected. In this context, said pocket area may be formed and/or accessible in such a manner that a proper placement of the terminals of the monitoring device 10 can be ensured with respect to the conductive pattern.

Moreover, the monitoring device 10 may advantageously comprise an insert part comprising the terminals. In this context, said insert part may especially be configured to be positioned inside the pocket. Additionally or alternatively, the monitoring device 10 may comprise a cover part being especially configured to not to be contained within the pocket and/or to be clamped against the insert part. In this context, especially sandwiching the respective layer with the conductive pattern in between the cover part and the insert part allows for locking the position of the terminals with respect to the conductive pattern.

It is further noted that it might be particularly advantageous if the acquired information comprises at least one measurement value of at least one of resistance, capacitance, inductance, impedance, or any combination thereof. In this context, for verifying the proper attachment of the monitoring device 10 to the diaper 14, the acquired information may especially comprise the result of a comparison between the at least one measurement value and at least one respective verification reference value, preferably at least one respective verification threshold value.

Furthermore, for identifying the type of the diaper 14, the acquired information may especially comprise the result of a comparison between the at least one measurement value and at least one respective identification reference value, preferably at least one respective identification threshold value, and/or the result of a type interpretation of the at least one measurement value. Moreover, for determining the wetness and/or saturation status of the diaper 14, the acquired information may especially comprise the result of a comparison between the at least one measurement value and at least one respective determination reference value, preferably at least one respective determination threshold value, and/or the result of a status interpretation of the at least one measurement value.

In addition to this or as an alternative, for verifying the proper attachment of the monitoring device 10 to the diaper 14, the processing unit 12 may especially be configured to select at least two, preferably at least two pairs, of the terminals being relatively widely spaced or having the greatest distance with regard to each other for acquiring the information. Further additionally or further alternatively, for verifying the proper attachment of the monitoring device 10 to the diaper 14, the processing unit 12 may especially be configured to select at least two pairs, preferably at least two, of the terminals 11a, 11b, 11c, 11d, 11e, 11f for acquiring the information in such a manner that the selected terminals are unambiguously shorted or left open by contacting the conductive pattern.

As it can further be seen from Fig. 1 the monitoring device 10 comprises a feedback unit 13 especially connected to the processing unit. In this context, the feedback unit 13 is configured to acoustically and/or optically and/or haptically notify a status with respect to at least one of the proper attachment of the monitoring device 10 to the diaper 14, the type of the diaper 14, the wetness and/or saturation of the diaper 14, or any combination thereof to a user. With respect to the diaper 14, it is noted that said diaper 14 in combination with the monitoring device 10 or the diaper monitoring device, respectively, may be called smart diaper. It is further noted that such a monitoring device or diaper monitoring device, respectively, may especially be embodied as a re-usable clip-on module.

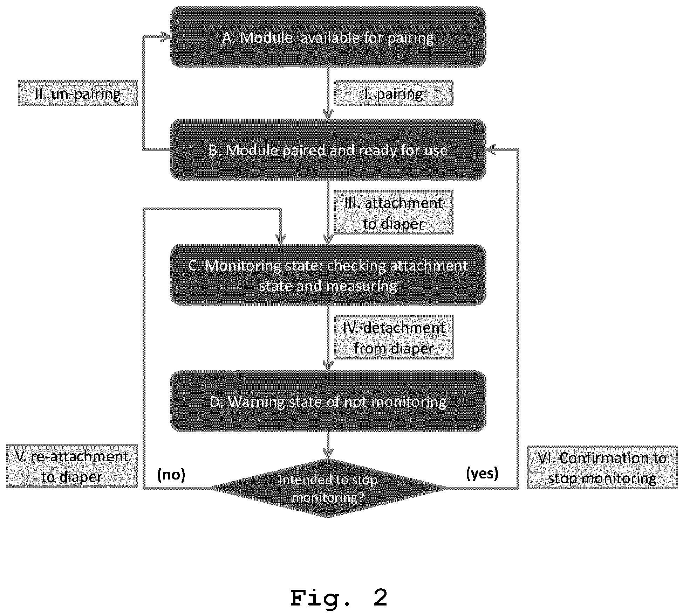

Accordingly, such a smart diaper in the sense of the invention especially combines a re-usable clip-on module with a disposable modified adult diaper that contains a sensor. By wearing the combination of the re-usable clip-on module and a disposable adult diaper, several measurement and sensor functionalities can be enabled, such as the monitoring of wetness inside the modified adult diaper through measuring impedance, capacitance or resistance values. Overall, the use of this system can be done as described in Fig. 2.

Said Fig. 2 shows a flow chart of a first exemplary embodiment of the inventive usage method for using the inventive monitoring device such as the monitoring device 10. In this context, it should be mentioned that it might be particularly advantageous if the monitoring devices or diaper monitoring devices, respectively, are intended to be used in a care home setting. In this setting, multiple subjects would simultaneously make use of the system.

Besides, multiple subjects would interchangeably make use of diaper monitoring devices, meaning that any devices could be linked to any subject's personal profile through a process especially called "pairing" (I. pairing). When such a device is paired to the subject, it can be attached to a diaper worn by this subject (III. attachment to diaper), allowing to go into the monitoring state.

When the device is detached from the diaper (IV. detachment from diaper), it can either be used again through re-attachment (V. re-attachment to diaper), or the removal may have taken place intentionally so that it can be confirmed (VI. Confirmation to stop monitoring). Upon being back in its state ready for being used, it can either be used again or it can be un-paired (II. un-pairing) upon which the device becomes available for being paired again to the same or to another subject. Any of the steps (I) to (VI) will further on be named with "event" (cf. "event message") above.

With respect to the above-mentioned pairing to another subject, it is noted that a step of cleaning may advantageously be advised before using the respective device with another person. Whereas the foregoing explanation of Fig. 2 is especially directed to the transitions between the different states, Fig. 2 will be explained in other words on the basis of the respective states in the following.

In accordance with Fig. 2, any device can typically be activated for use (status A), after which it may be paired to a person's personal identifier (I. Pairing). The step of pairing, as already mentioned above, especially involves creating a link between the device and the person's personal identifier, that allows for assigning data that will be sent by this respective device automatically to that person's profile, and that will add the person's details (preferably, the person's name, initials, location of residence, ...) towards a caregiver upon receiving a notification about the person's status.

After pairing (I.), the respective device is paired and ready for use according to state B. In this context, a step of un-pairing (II.) would lead from state B to the above-mentioned state A. When a device is paired and ready for use (status B), it can be attached to a diaper (III.) which will automatically be recognized by the monitoring device and which will activate the monitoring of the device (sending data especially wirelessly, for updating the person's information and storing it, and for notifying a caregiver about status changes). Accordingly, the respective device is in monitoring state (C.), which especially comprises checking attachment state and performing measurements with respect to the diaper and/or the wearer of the diaper.

Moreover, a detachment from the diaper (IV.) would lead to a warning state (D.) of not monitoring. In the case that it is not intended to stop monitoring, the respective device should be re-attached to the diaper (V.). Further interaction such as the stopping of monitoring (VI.) or a confirmation thereof, respectively, is also enabled. As already mentioned above, unpairing of a device is also possible, and removes the link between the person's personal identifier and the device - making the device available to be paired again to the same person's or to another person's personal identifier.

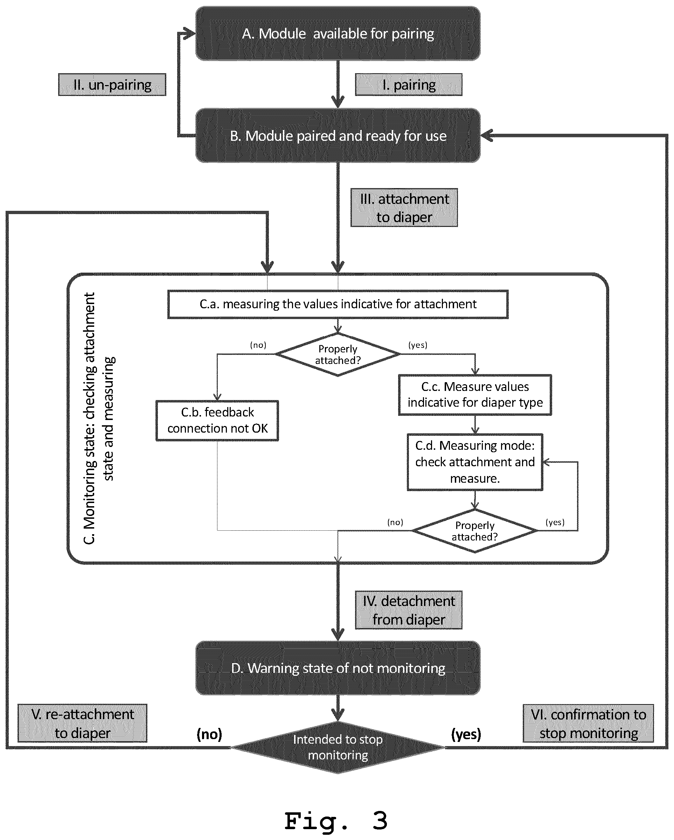

For what can happen during the monitoring state of a paired diaper monitoring device that is in use for the application of monitoring, especially wetness monitoring, within a diaper, an overview is given in detail within Fig. 3. More specifically, Fig. 3 illustrates in particular the steps that are related to verification of proper diaper attachment, identifying a diaper type and measuring values indicative for exudates within a modified diaper such as the diaper 14 of Fig. 1.

In this context, the steps under 'C. monitoring state: checking attachment state and measuring' are now described further in detail, for an exemplary case with n terminals of the monitoring device and a corresponding conductive pattern or a printed sensor design, respectively, especially within a pocket area of the absorbent article or of the diaper, respectively. In accordance with the step of attachment verification (C.a), it is noted that whenever a monitoring device or clip-on module is not connected to a diaper's conductive pattern or print, it is not measuring.

Furthermore, attachment to a diaper (III) and herewith making connection to a conductive pattern or print, will activate the measuring status of the monitoring device or clip-on module. Upon initial connection, it will verify whether the connection to the pattern or print is made in a proper way, through a series of n logic steps:

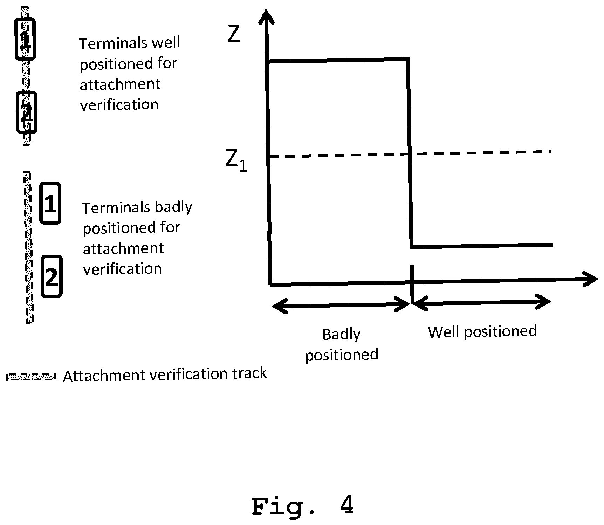

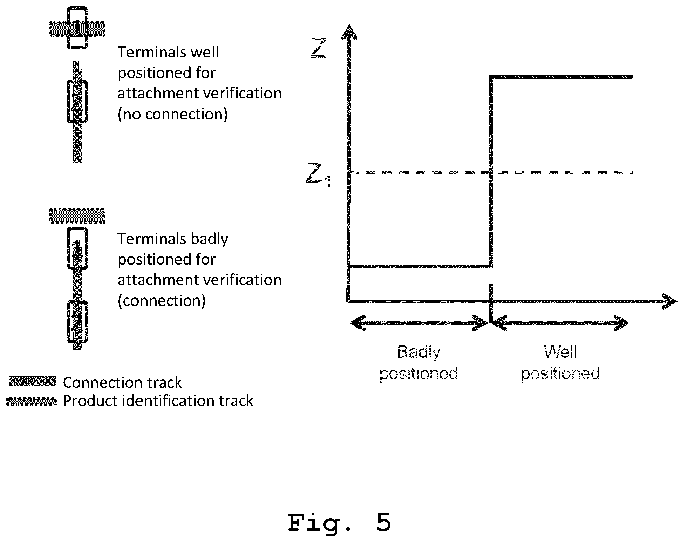

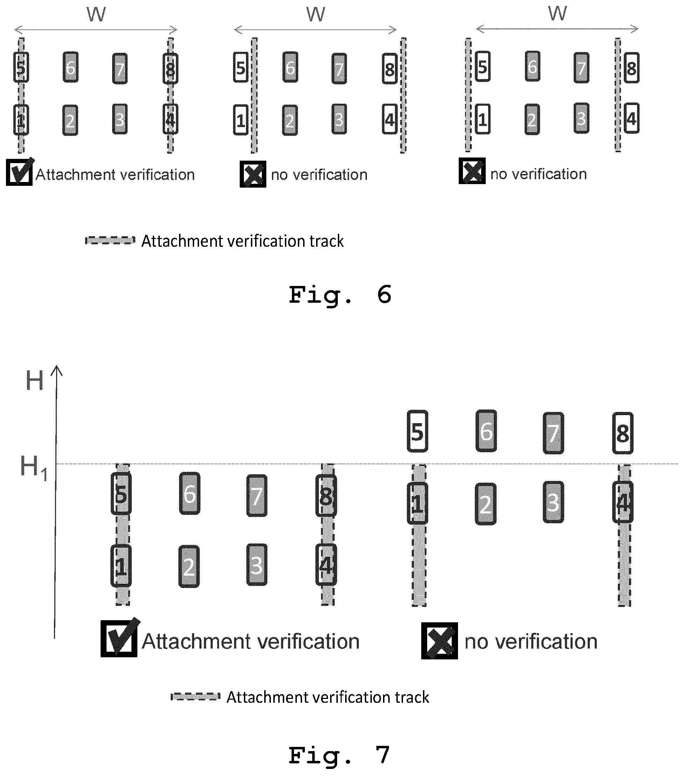

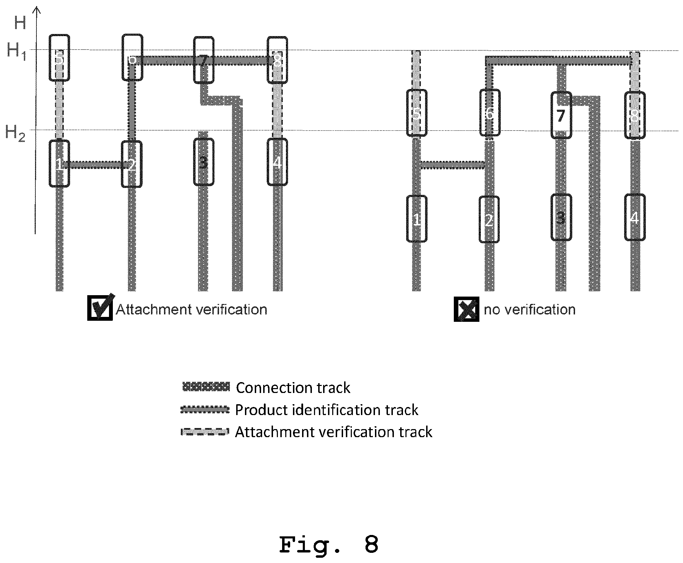

For a select amount of combinations between i and j, with i ranging within a selection amongst terminals 1 to n, and j ranging within a selection amongst terminals 1 to n, the condition will be checked: "if the resistance between terminal i and terminal j is below the specified threshold, the condition is accepted" (see Fig. 4 or Fig. 7), or alternatively "if the resistance between terminal i and terminal j is above the specified threshold, the condition is accepted" (see Fig. 5 or Fig. 8). The select amount of combinations can address a set of attachment verification terminals and/or combined purpose terminals.

According to step C.b, when the condition was not accepted, the monitoring device or clip-on can notify the user about this through auditive and/or visual and/or haptic feedback towards the user, to encourage repeating attachment to a diaper (III). Additionally or alternatively, the lacking of a foreseen positive feedback of proper attachment, may encourage repeating attachment to a diaper. Furthermore, in accordance with the step of product identification (C.c), if attachment verification has been found according to step C.a, the device or clip-on will move over to making the reading for interpretation of the product identification.

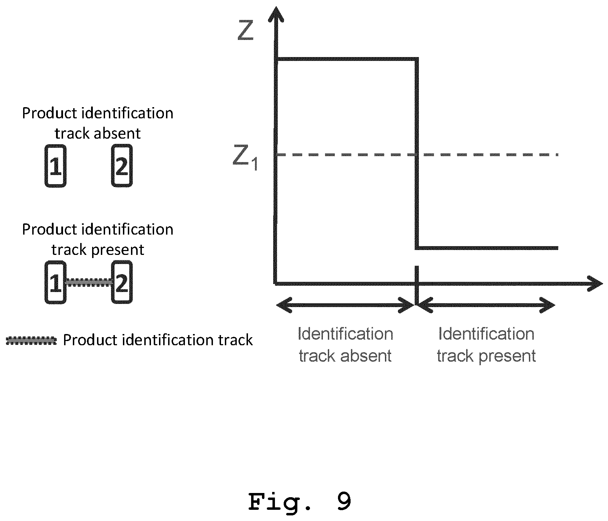

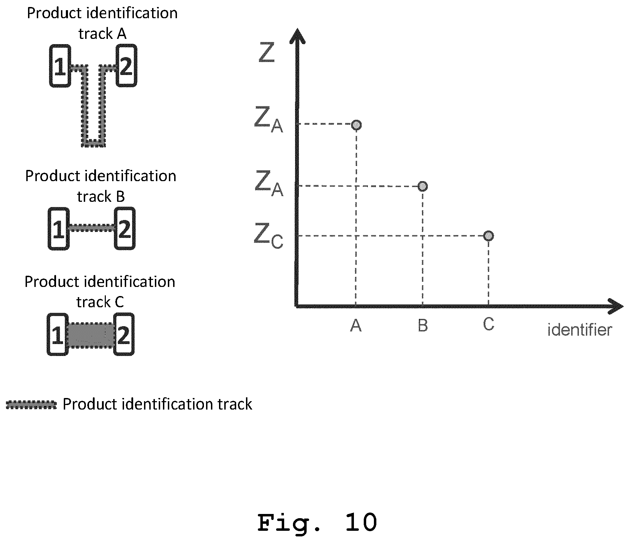

For a selected amount of combinations between i and j (list [X]), with i ranging within a selection amongst terminals 1 to n, and j ranging within a selection amongst terminals 1 to n, a bit will be assigned: "if the resistance between terminal i and terminal j is below the specified threshold, its bit will especially be interpreted as 1; else, it will especially be interpreted as 0" (see Fig. 9). As an alternative to the threshold, a value can be measured (see Fig. 10). The selected amount of combinations can address a set of product identification terminals and/or combined purpose terminals.

It is noted that it might be particularly advantageous if the data resulting from this list of measurements, will be transmitted to a cloud application. This list can then be interpreted by the cloud application, because each possible combination of values for the list of terminal combinations i and j (list [X]), can be related to a specific diaper type (the absorbent product type identifier).

It is further noted that a combination of multiple connections that the monitoring device can make with the plurality of terminals to the conductive pattern may preferably result in a permutation with a respective number of product types, i.e. with open and closed connections between the terminals especially of the above-mentioned matrix.

In accordance with the step of monitoring exudates inside the diaper (C.d), after having transmitted the information about the product identifier according to step C.c, the monitoring device or clip-on module, respectively, will be in measurement mode.

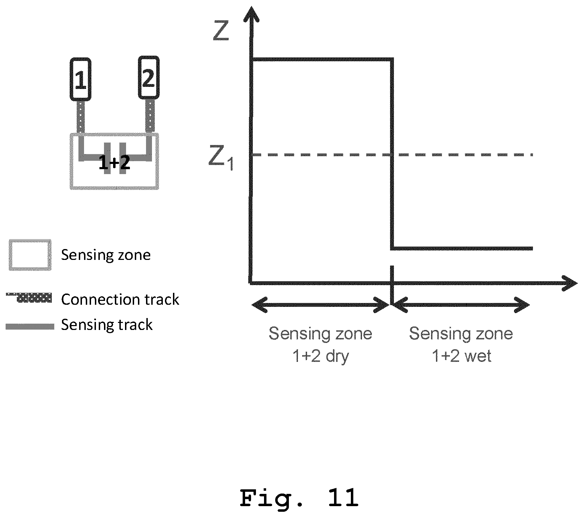

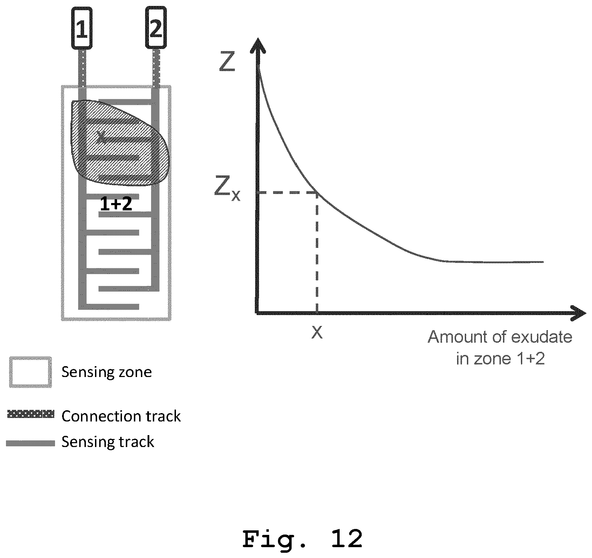

It will perform a reading every defined time interval *tₘₑₐₛᵤᵣₑ.* For a selected amount of combinations between i and j (list [Y]), with i ranging within a selection amongst terminals 1 to n, and j ranging within a selection amongst terminals 1 to n, a bit will be assigned: "if the resistance between terminal i and terminal j is below the specified threshold, its bit will especially be interpreted as 1; else, it will especially be interpreted as 0" (threshold approach for measurement terminals, see Fig. 11). As an alternative to the threshold, a value can be measured (value measurement approach for measurement, see Fig. 12). The select amount of combinations can address a set of measurement terminals and/or combined purpose terminals.

It is noted that it might be particularly advantageous if the respective series of bits resulting from this measurement will be transmitted to a cloud application such as the above-mentioned cloud application if there was a change versus the previous measurement. Furthermore, if meanwhile the values indicative for proper attachment as considered in step C.a have changed, notification will especially be raised if attachment is not good or proper anymore. Moreover, if no change has been observed for a predefined amount of time (and thus after a pre-defined amount of measurements, or after a pre-defined multiple of *tₘₑₐₛᵤᵣₑ* ), it will automatically send the non-changed information as well to provide information to the cloud about if the device or clip-on is still within the network's connectivity range. This array of bits can then be interpreted by the cloud application, because each possible combination of values for the list of terminal combinations i and j (list [Y]), can be related to a possible status of saturation within the absorbent core of the diaper.

Now, with respect to the attachment verification according to step C.a, an exemplary embodiment thereof is shown in Fig. 4. In this context, a measurement for attachment verification can be executed by comparing a measured value to a threshold value (which may be built-in to the monitoring device, preferably in the firmware thereof), where the defined threshold may be related to the presence of a certain connection (presence type). As exemplary explained in Fig. 4, wherein Z might stand for resistance; for values of Z > Z1, value "0" would especially be assigned to the combination of measurement terminals 1 and 2 (badly positioned, terminals not in the right place versus the conductive pattern or print); for values of Z < Z1, value "1" would especially be assigned to the combination of measurement terminals 1 and 2 (well positioned).

As an alternative to said presence type, Fig. 5 illustrates an exemplary embodiment in the sense of an absence type.

As it can be seen, a reading for attachment verification can be executed by comparing a measured value to a threshold value (which may be built-in to the monitoring device, preferably in the respective firmware thereof), wherein the defined threshold may be related to the absence of a certain connection. As exemplary explained in Fig. 5, wherein Z might stand for resistance; for values of Z < Z1, value "0" would especially be assigned to the combination of measurement terminals 1 and 2 (badly positioned, terminals placed on a track where not intended to be placed on); for values of Z > Z1, value "1" would especially be assigned to the combination of measurement terminals 1 and 2 (well positioned, not connected where not intended to be connected).

As a further embodiment, for a certain arrangement of terminals on a certain width W, attachment verification tracks are applied at the respective extreme sides of the beginning and end of W. This allows to detect any possibly bad positioning both before the beginning of W and after the end of W. As illustrated in Fig. 6, this especially has the advantage that a control measure is built in to determine if terminals are not positioned too much left or right on the width W. If the distance between all terminals is fixed (as they are incorporated into the monitoring device or clip-on module, respectively), this therefore ensures that the terminals in between are also positioned well alongside W. In the example of Fig. 6, the attachment verification would especially be made by checking if the measurement between terminals 1 and 5 or between terminals 4 and 8, respectively, results in a value below the specified threshold (presence of the tracks).

As a further exemplary embodiment, for a certain arrangement of terminals along height H, attachment verification tracks stop on a certain height H1 according to Fig. 7. This especially allows avoiding any possibly bad positioning above H1 which would not result in attachment verification. As illustrated by Fig. 7, this has the advantage that a control measure is built in to determine if terminals are not positioned too high along the height H on the conductive pattern or print, which could result in erroneous measurement and identifier interpretations. In the example of Fig. 7, the attachment verification would be made by checking if the measurement between terminals 1 and 5 or between terminals 4 and 8, respectively, results in a value below the specified threshold (presence of the tracks).

As a further exemplary embodiment, for a certain arrangement of terminals along height H, one or several of any tracks (for example connection tracks) stop on a certain height H2 below H1 according to Fig. 8. As such, the check if no connection is present can especially be included in the list of attachment verification checks. As illustrated in Fig. 8, this especially has the advantage that a control measure is built in to determine if terminals are not positioned too low along the height H on the conductive pattern or print, which could result in erroneous measurement and identifier interpretations. In the example of Fig. 8, the attachment verification would be made by checking if the measurement between terminals 3 and 7 results in a value above the specified threshold (absence of a connection).

Generally, it might be particularly advantageous if some terminals are positioned a bit higher versus the printed tracks than others versus the printed tracks; the ones which are higher would serve for attachment verification (in contact = verification of attachment), whereas others would serve for product identification or measurement (in contact = able to measure), but by placing those at a position where the overlap from terminal to print will always be bigger than the overlap from an attachment verification terminal to a printed track, safety is built in for correct product identification and exudate monitoring measurements.

Exemplarily, in accordance with Fig. 8, attachment verification terminal 5 and combined purpose terminal 8 (that connects to an attachment verification track) are placed higher versus the print than the other terminals that serve for product identification and measurement. Alternatively, for a given arrangement of terminals, the attachment verification tracks could have been made shorter at the top to achieve the same relative result.

This especially has the advantage that even upon attachment verification where terminals are only just in contact to the attachment verification tracks, there may already be a sufficient overlap from other terminals to connection tracks or product identification tracks. A lower contact area could have higher chance of resulting into erroneous reading (less efficient contact, resulting in not reaching the conductivity threshold).

With respect to the above-mentioned step of product identification according to step C.c, a measurement for identification can be executed by comparing a measured value to a threshold value (which may be built-in to the monitoring device, preferably in the respective firmware thereof). As exemplary illustrated by Fig. 9, wherein Z might stand for resistance; for values of Z > Z1, value "0" would especially be assigned to the combination of measurement terminals 1 and 2 (product identification track absent); for values of Z < Z1, value "1" would especially be assigned to the combination of measurement terminals 1 and 2 (product identification track present).

As an alternative embodiment, a measurement for identification can be executed by interpreting any measured value. As exemplary explained in Fig. 10, wherein Z might stand for resistance; the value of Z might be characteristic for the specific identifier, for instance, Z might take on value Z_{A} for a product identification track A, Z_{B} for a product identification track B, or Z_{C} for a product identification track C.

Now, with respect to the above-mentioned exudate monitoring according to step C.d, a measurement for exudate monitoring can be executed by comparing a measured value to a threshold value (which may be built-in to the monitoring device, preferably in the respective firmware thereof). As exemplary explained in Fig. 11, wherein Z might stand for resistance and the exudate might be liquid absorbed into the product's absorbent core; for values of Z > Z1, value "0" would especially be assigned to the combination of measurement terminals 1 and 2 (dry); for values of Z < Z1, value "1" would especially be assigned to the combination of measurement terminals 1 and 2 (wet).

As a further exemplary embodiment, a measurement for exudate monitoring can be executed by interpreting any measured value. As exemplary illustrated by Fig. 12, wherein Z might stand for resistance and the exudate might be liquid absorbed into the product's absorbent core; the value of Z might change depending on the amount of exudate within "zone 1+2", for instance, Z might take on value Z_{X} for an amount of exudate X.

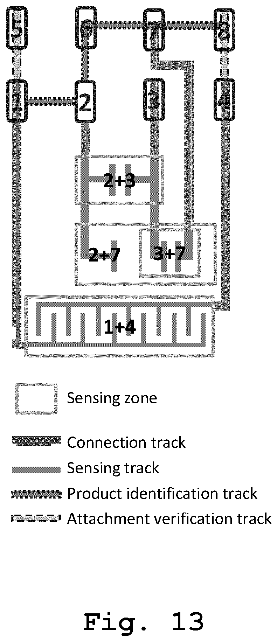

Furthermore, Fig. 13 shows an explanatory example case with eight terminals. Said example case or exemplary embodiment, respectively, comprises the following characteristics:
- Terminal 3 is a measurement terminal (can only connect to a connection track);
- Terminals 6 is a product identification terminal (can only connect to product identification tracks);
- Terminal 5 is an attachment verification terminal (can only connect to an attachment verification track); and
- Terminals 1, 2, 4, 7 and 8 are combined purpose terminals (can connect to connection track and/or product identification track and/or attachment verification track).

As a parenthesis, the foregoing terms will be explained in the following in greater detail. Additionally, some further explanations will be made:
- Conductive print: a certain pattern of conductive ink that is preferably applied to the inner side of an absorbent article's backsheet. Parts of the pattern are typically referred to as "tracks", and a track may have any shape but it typically has a length and a width, wherein the length may preferably be much longer than the width;
- Monitoring device or clip-on module or clip-on data processing module: a device that can be attached onto a modified absorbent article (modified in that it may comprise an opening to insert the module there through and a pocket to retain the module therein);
- Terminals: parts on a clip-on data processing module, preferably metal parts or parts of a highly conductive material, that are coupled to electronics which may be incorporated into the module such as the processing unit 12 of Fig. 1;
- Connection tracks: parts of a conductive print that foresee an electrical connection from certain elements (e.g. sensing tracks) to a location where they can be brought into electrical connection with terminals;
- Attachment verification terminals: terminals that serve only for attachment verification;
- Attachment verification tracks: parts of a conductive print that may be elongations of other tracks, typically of connection tracks, and to which attachment verification terminals can be brought into electrical connection;
- Sensing tracks: parts of a conductive print that undergo changes (e.g. because of where they are located) when exudates arrive into the absorbent article, for instance, urine which is absorbed by the absorbent article's core;
- Sensing zones: zones of an absorbent core, of which changes can be interpreted by certain sensing tracks;
- Measurement terminals: terminals that serve only for measuring (e.g. for measuring changes that have occurred along sensing tracks);
- Product identification terminals: terminals that serve only for product identification;
- Product identification tracks: parts of a conductive print, that may be elongations of or connections between other tracks (typically of or between connection tracks), and to which product identification terminals can be brought into electrical interconnection; and
- Combined purpose terminals: terminals that can serve for attachment verification and/or for measurements and/or for product identification. They may connect to connection tracks which are typically elongated with tracks that are either product identification tracks or attachment verification tracks.

With respect to all the explanations and/or definitions above, it is to be pointed out that each of the foregoing terms is not limited thereto.

Again, with respect to the explanatory example case with eight terminals according to Fig. 13, for this case, the steps of Fig. 3 would exemplarily address the following terminal combinations:
- For attachment verification (C.a): measurement between 1-5 and 4-8, disconnection of 7-3;
- For product identification (C.c): measurements between [1-2, 2-6, 6-7, 7-8]. (list [X]); and
- For exudate monitoring (C.d): measurements between [2-3, 2-7, 3-7, 1-4]. (list [Y]).

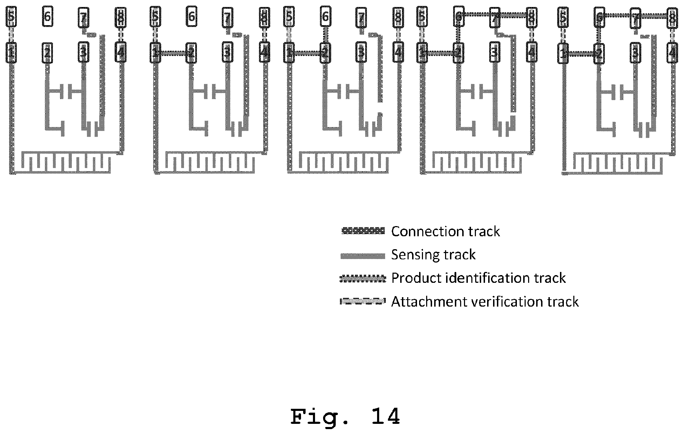

With this explanatory case according to Fig. 13, several product identifiers or absorbent product type identifiers, respectively, can be constructed, and applied to different types of absorbent products (using the same or a different pattern of sensing tracks, but preferably using the same pattern for attachment verification tracks). A non-exhaustive set from the total of 16 available product identification options of this example is shown in Fig. 14.

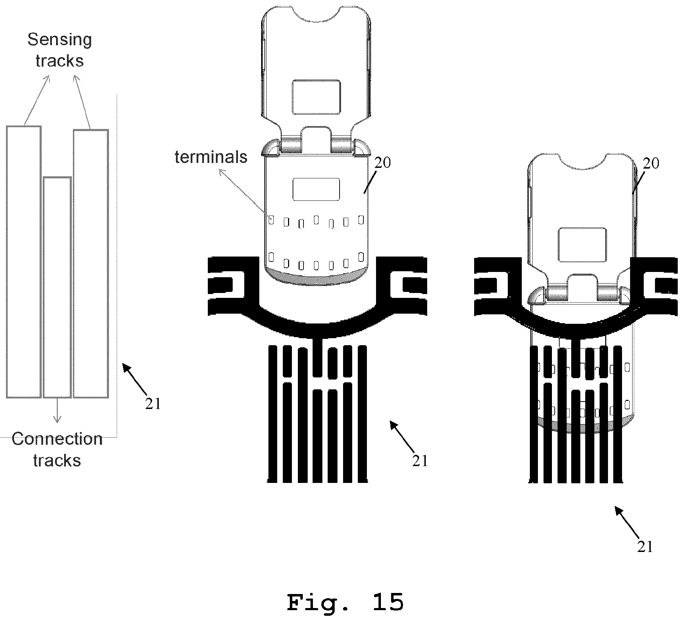

Now, with respect to Fig. 15, an exemplary product design of the monitoring device 20 with 14 terminals and a corresponding design of a conductive pattern 21 are illustrated. In this context, it should be noted that there can be an overlap between elements (combinations of i and j) from list [X] and list [Y], in which case a series of logical steps especially within the cloud can determine whether a value 0 or a value 1 can be accounted to a connection specific for a diaper product type ID, or a connection specific for a wet zone within the absorbent core.

Furthermore, rules can be defined for determining the available product identification track arrangement for a given arrangement of attachment verification tracks, connection tracks and sensing tracks.

It should furthermore be noted that several variations can be developed for performed measurements from the monitoring device or the clip-on module, respectively; these measurements may especially be capacitance, impedance or resistance values.

For attachment verification measurements, these may preferably be resistance measurements that are compared to the clip-on module's built in threshold values.

For product identification measurements, these may preferably be resistance measurements that are compared to the clip-on module's built in threshold values.

For measurements that relate to sensing tracks, these may preferably be resistance measurements that are compared to the clip-on module's built in threshold values.

Alternatively, resistance, capacitance or impedance measurements can be performed, where specific values are associated to specific conditions of soiling in the absorbent article.

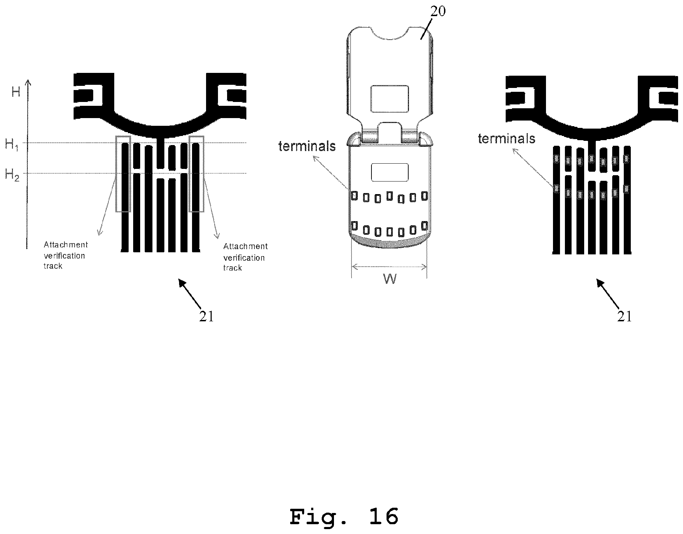

As a further exemplary embodiment, especially a preferred embodiment of Fig. 15, for a certain arrangement of terminals along height H, one or several of any tracks (for example connection tracks) stop on a certain height H2 below H1 according to Fig. 16. As such, the check if no connection is present can especially be included in the list of attachment verification checks.

As illustrated in Fig. 16, this especially has the advantage that a control measure is built in to determine if terminals are not positioned too low along the height H on the conductive pattern or print, which could result in erroneous measurement and identifier interpretations. As it can further be seen from Fig. 16, for a certain arrangement of terminals on a certain width W, attachment verification tracks are applied at the respective extreme sides of the beginning and end of W.

This especially allows to detect any possibly bad positioning both before the beginning of W and after the end of W. As illustrated in Fig. 16, this especially has the advantage that a control measure is built in to determine if terminals are not positioned too much left or right on the width W. If the distance between all terminals is fixed (as they are incorporated into the monitoring device or clip-on module, respectively), this therefore ensures that the terminals in between are also positioned well alongside W.

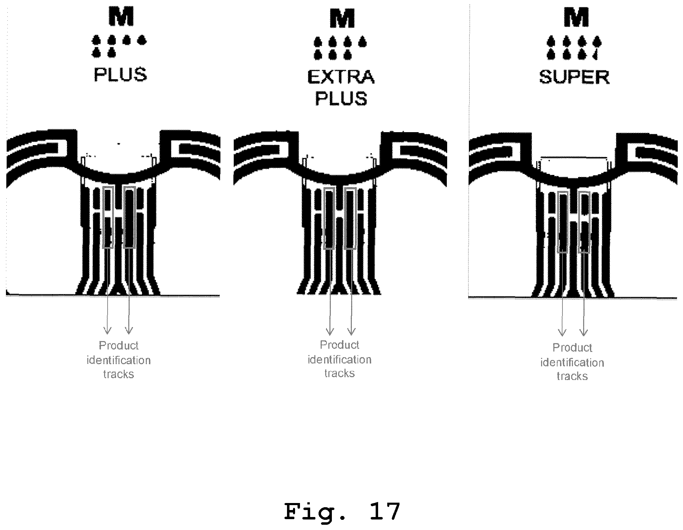

Moreover, Fig. 17 shows three exemplary embodiments of the conductive pattern such as the above-mentioned conductive pattern 21 with special respect to different product identification tracks. In this context, the respective absorbing capacity exemplarily increases from the left embodiment to the right embodiment.

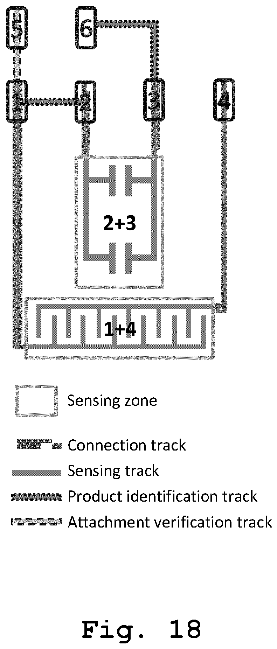

Furthermore, Fig. 18 shows an explanatory example case with six terminals. Said example case or exemplary embodiment, respectively, comprises the following characteristics:
- Terminal 4 is a measurement terminal (can only connect to a connection track);
- Terminals 6 is a product identification terminal (can only connect to product identification tracks);
- Terminal 5 is an attachment verification terminal (can only connect to an attachment verification track); and
- Terminals 1, 2, and 3 are combined purpose terminals (can connect to connection track and/or product identification track and/or attachment verification track).

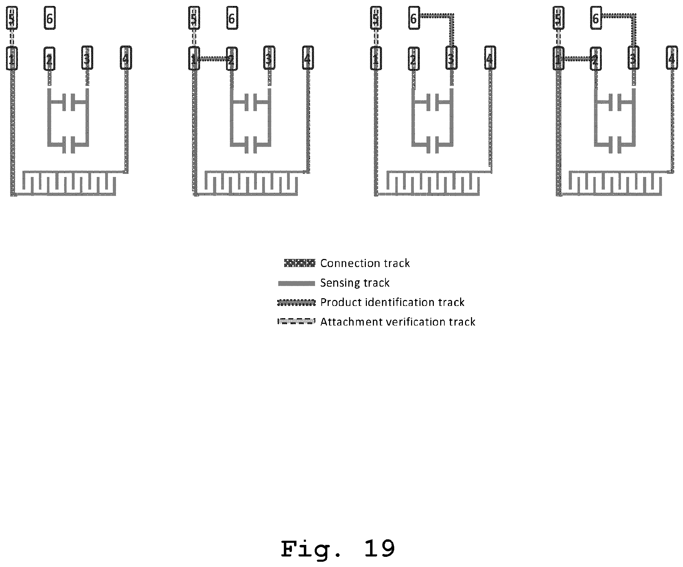

With this explanatory case according to Fig. 18, several product identifiers or absorbent product type identifiers, respectively, can be constructed, and applied to different types of absorbent products (using the same or a different pattern of sensing tracks, but preferably using the same pattern for attachment verification tracks). A set of four product identification options of this example is shown in Fig. 19.

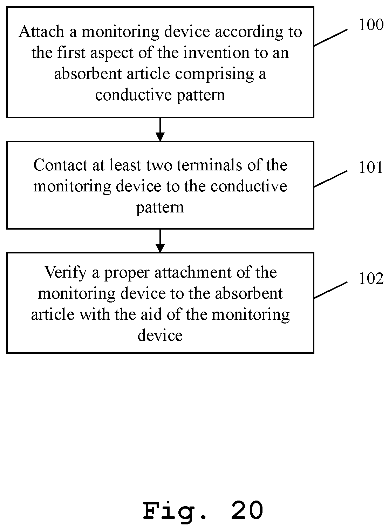

Finally, Fig. 20 shows a flow chart of a second exemplary embodiment of the inventive method for using a monitoring device such as the above-mentioned monitoring device 10. According to a first step 100, said usage method comprises the step of attaching the monitoring device to an absorbent article comprising a conductive pattern. Then, in a second step 101, the method comprises contacting the at least two terminals, exemplarily the six terminals 11a, 11b, 11c, 11d, 11e, 11f, of the monitoring device to the conductive pattern. Additionally, the usage method comprises verifying a proper attachment of the monitoring device to the absorbent article such as the diaper 14 with the aid of the monitoring device.

It might be particularly advantageous if the usage method comprises the step of repeating or correcting the attachment of the monitoring device to the absorbent article in the case of an improper attachment until the monitoring device is properly attached to the absorbent article. In addition to this or as an alternative, the method may comprise the step of identifying the type of the absorbent article in the case of a proper attachment with the aid of the monitoring device.

Furthermore, the usage method may comprise the step of determining the wetness and/or saturation status of the absorbent article with the aid of the monitoring device especially on the basis of the type of the absorbent article.

While various embodiments of the present invention have been described above, it should be understood that they have been presented by way of example only, and not limitation. Numerous changes to the disclosed embodiments can be made in accordance with the disclosure herein without departing from the scope of the invention. Thus, the breadth and scope of the present invention should not be limited by any of the above described embodiments. Rather, the scope of the invention should be defined in accordance with the following claims

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A monitoring device (10) attachable to an absorbent article (14) comprising a conductive pattern, the monitoring device (10) comprising:
at least three terminals (11a, 11b, 11c) contactable to the conductive pattern, wherein the terminals (11a, 11b, 11c) are arranged on the monitoring device (10) according to a predefined terminal pattern, and wherein the predefined terminal pattern comprises or is a matrix; and
a processing unit (12) connected to the at least three terminals (11a, 11b, 11c),
wherein, in the case that the monitoring device (10) is attached to the absorbent article (14) and the at least three terminals (11a, 11b, 11c) contact the conductive pattern at least partly, on the basis of the contact between at least a part of the at least three terminals (11a, 11b, 11c) and the conductive pattern, the processing unit (12) is configured to acquire information for verifying a proper attachment of the monitoring device (10) to the absorbent article (14) and/or for identifying a type of the absorbent article (14) and/or for determining a wetness and/or saturation status of the absorbent article (14), wherein the acquired information comprises at least one measurement value of at least one of resistance, capacitance, inductance, impedance, or any combination thereof

2. The monitoring device (10) according to claim 1, wherein the monitoring device (10) comprises at least four terminals, preferably six terminals, more preferably eight terminals, most preferably fourteen terminals.

3. The monitoring device (10) according to claim 1 or 2, wherein the distance between the terminals with regard to each other is predefined.

4. The monitoring device (10) according to claim 3,
wherein the matrix comprises two rows and/or two columns.

5. The monitoring device (10) according to any of the claims 1 to 4,
wherein for verifying the proper attachment of the monitoring device (10) to the absorbent article (14), the acquired information comprises the result of a comparison between the at least one measurement value and at least one respective verification reference value, preferably at least one respective verification threshold value.

6. The monitoring device (10) according to any of the claims 1 to 5,
wherein for identifying the type of the absorbent article (14), the acquired information comprises the result of a comparison between the at least one measurement value and at least one respective identification reference value, preferably at least one respective identification threshold value, and/or the result of a type interpretation of the at least one measurement value.

7. The monitoring device (10) according to any of the claims 1 to 6,
wherein for determining the wetness and/or saturation status of the absorbent article (14), the acquired information comprises the result of a comparison between the at least one measurement value and at least one respective determination reference value, preferably at least one respective determination threshold value, and/or the result of a status interpretation of the at least one measurement value.

8. The monitoring device (10) according to any of the claims 1 to 7,
wherein for verifying the proper attachment of the monitoring device (10) to the absorbent article (14), the processing unit (12) is configured to select at least two, preferably at least two pairs, of the terminals being relatively widely spaced or having the greatest distance with regard to each other for acquiring the information.

9. The monitoring device (10) according to any of the claims 1 to 7,
wherein for verifying the proper attachment of the monitoring device (10) to the absorbent article (14) and/or for identifying the type of the absorbent article (14), the processing unit (12) is configured to select at least two, preferably at least two pairs, of the terminals for acquiring the information in such a manner that the selected terminals are unambiguously shorted or left open by contacting the conductive pattern.

10. The monitoring device (10) according to any of the claims 1 to 9,
wherein the monitoring device (10) comprises a feedback unit (13),
wherein the feedback unit (13) is configured to acoustically and/or optically and/or haptically notify a status with respect to at least one of the proper attachment of the monitoring device (10) to the absorbent article (14), the type of the absorbent article (14), the wetness and/or saturation of the absorbent article (14), or any combination thereof to a user.

11. A method for using a monitoring device (10) according to any of the claims 1 to 10, the method comprising the steps of:
attaching the monitoring device (10) to an absorbent article (14) comprising a conductive pattern,
contacting the at least three terminals (11a, 11b, 11c) of the monitoring device (10) to the conductive pattern, and
verifying a proper attachment of the monitoring device (10) to the absorbent article (14) with the aid of the monitoring device (10).

12. The method according to claim 11,
wherein the method comprises the steps of:
repeating or correcting the attachment of the monitoring device (10) to the absorbent article (14) in the case of an improper attachment until the monitoring device (10) is properly attached to the absorbent article (14), and/or
identifying the type of the absorbent article (14) in the case of a proper attachment with the aid of the monitoring device (10).

13. The method according to claim 12,
wherein the method comprises the step of determining the wetness and/or saturation status of the absorbent article (14) with the aid of the monitoring device (10) especially on the basis of the type of the absorbent article (14) and/or especially only after having verified the proper attachment of the monitoring device to the absorbent article.

14. An absorbent article (14) comprising:
a conductive pattern (21) and a monitoring device (10, 20) as defined in Claim 1
wherein the conductive pattern (21) comprises conductive tracks,
wherein the conductive tracks are formed in such a manner that the conductive tracks interconnect at least three terminals (11a, 11b, 11c) of said monitoring device (10, 20) according to a predefined pattern if the conductive pattern (21) is contacted by the at least three terminals (11a, 11b, 11c) of the monitoring device (10, 20).

15. The absorbent article (14) according to claim 14,
wherein the conductive pattern (21) comprises an ink, preferably a carbon-based ink and/or a conductive polymer-based ink.

16. The absorbent article (14) according to claim 15, wherein the ink comprises at least one of graphene, graphite, nano-carbon-tubes, polyacetylene, polypyrrole, polyaniline and copolymers thereof, poly(pyrrole)s (PPY), polyanilines (PANI), poly(thiophene)s (PT), poly(p-phenylene sulfide) (PPS), poly(p-phenylene) (PPP), Poly (acetylene) s (PAC), Poly (p-phenylene vinylene) (PPV), poly(3,4-ethylenedioxythiophene) (PEDOT), poly(3,4-ethylenedioxythiophene) polystyrene sulfonate (PEDOT:PSS), or any combination thereof.

## Patentansprüche

1. Überwachungsvorrichtung (10), die an einem saugfähigen Artikel (14) befestigt werden kann, der ein Leiterbild umfasst, wobei die Überwachungsvorrichtung (10) umfasst:
mindestens drei mit dem Leiterbild kontaktierbare Anschlüsse (11a, 11b, 11c), wobei die Anschlüsse (11a, 11b, 11c) auf der Überwachungsvorrichtung (10) gemäß einem vordefinierten Anschlussmuster angeordnet sind und wobei das vordefinierte Anschlussmuster eine Matrix umfasst oder ist; und
eine mit den mindestens drei Anschlüssen (11a, 11b, 11c) verbundene Verarbeitungseinheit (12),
wobei in dem Fall, dass die Überwachungsvorrichtung (10) an dem saugfähigen Artikel (14) angebracht ist und die mindestens drei Anschlüsse (11a, 11b, 11c) das Leiterbild zumindest teilweise berühren, die Verarbeitungseinheit (12) auf der Grundlage des Kontakts zwischen mindestens einem Teil der mindestens drei Anschlüsse (11a, 11b, 11c) und dem Leiterbild konfiguriert ist, um Informationen zum Verifizieren einer ordnungsgemäßen Anbringung der Überwachungsvorrichtung (10) an dem saugfähigen Artikel (14) und/oder zum Identifizieren eines Typs des saugfähigen Artikels (14) und/oder zum Bestimmen eines Nässe- und/oder Sättigungszustands des saugfähigen Artikels (14) zu erfassen, wobei die erfassten Informationen mindestens einen Messwert von mindestens einem von Widerstand, Kapazität, Induktivität, Impedanz oder einer beliebigen Kombination davon umfasst

2. Überwachungsvorrichtung (10) nach Anspruch 1, wobei die Überwachungsvorrichtung (10) mindestens vier Anschlüsse, vorzugsweise sechs Anschlüsse, mehr bevorzugt acht Anschlüsse, am meisten bevorzugt vierzehn Anschlüsse umfasst.

3. Überwachungsvorrichtung (10) nach Anspruch 1 oder 2, wobei der Abstand zwischen den Anschlüssen untereinander vordefiniert ist.

4. Überwachungsvorrichtung (10) nach Anspruch 3, wobei die Matrix zwei Zeilen und/oder zwei Spalten umfasst.

5. Überwachungsvorrichtung (10) nach einem der Ansprüche 1 bis 4,
wobei zum Überprüfen der ordnungsgemäßen Anbringung der Überwachungsvorrichtung (10) an dem saugfähigen Artikel (14) die erfassten Informationen das Ergebnis eines Vergleichs zwischen dem mindestens einen Messwert und mindestens einem jeweiligen Überprüfungsreferenzwert, vorzugsweise mindestens einem jeweiligen Überprüfungsschwellenwert, umfassen.

6. Überwachungsvorrichtung (10) nach einem der Ansprüche 1 bis 5,
wobei zum Identifizieren des Typs des saugfähigen Artikels (14) die erfassten Informationen das Ergebnis eines Vergleichs zwischen dem mindestens einen Messwert und mindestens einem jeweiligen Identifizierungsreferenzwert, vorzugsweise mindestens einem jeweiligen Identifizierungsschwellenwert, und/oder das Ergebnis einer Typinterpretation des mindestens einen Messwerts umfassen.

7. Überwachungsvorrichtung (10) nach einem der Ansprüche 1 bis 6,
wobei zum Bestimmen des Nässe- und/oder Sättigungszustands des saugfähigen Artikels (14) die erfassten Informationen das Ergebnis eines Vergleichs zwischen dem mindestens einen Messwert und mindestens einem jeweiligen Bestimmungsreferenzwert, vorzugsweise mindestens einem jeweiligen Bestimmungsschwellenwert, und/oder das Ergebnis einer Zustandsinterpretation des mindestens einen Messwerts umfassen.

8. Überwachungsvorrichtung (10) nach einem der Ansprüche 1 bis 7,
wobei die Verarbeitungseinheit (12) zum Überprüfen der ordnungsgemäßen Anbringung der Überwachungsvorrichtung (10) an dem saugfähigen Artikel (14) konfiguriert ist, um mindestens zwei, vorzugsweise mindestens zwei Paare, der Anschlüsse, die relativ weit beabstandet sind oder den größten Abstand bezüglich einander aufweisen, zum Erfassen der Informationen auswählt.

9. Überwachungsvorrichtung (10) nach einem der Ansprüche 1 bis 7,
wobei zum Überprüfen der ordnungsgemäßen Anbringung der Überwachungsvorrichtung (10) an dem saugfähigen Artikel (14) und/oder zum Identifizieren des Typs des saugfähigen Artikels (14) die Verarbeitungseinheit (12) konfiguriert ist, mindestens zwei, vorzugsweise mindestens zwei Paare, der Anschlüsse zum Erfassen der Informationen derart auszuwählen, dass die ausgewählten Anschlüsse durch Kontaktieren des Leiterbilds eindeutig kurzgeschlossen oder offen gelassen werden.

10. Überwachungsvorrichtung (10) nach einem der Ansprüche 1 bis 9,
wobei die Überwachungsvorrichtung (10) eine Rückmeldeeinheit (13) umfasst,
wobei die Rückmeldungseinheit (13) konfiguriert ist, um einen Benutzer über einen Zustand hinsichtlich mindestens eines von der ordnungsgemäßen Anbringung der Überwachungsvorrichtung (10) am saugfähigen Artikel (14), dem Typ des saugfähigen Artikels (14), der Nässe und/oder Sättigung des saugfähigen Artikels (14) oder eine beliebige Kombination davon akustisch und/oder optisch und/oder haptisch zu informieren.

11. Verfahren zum Verwenden einer Überwachungsvorrichtung (10) nach einem der Ansprüche 1 bis 10, wobei das Verfahren die folgenden Schritte umfasst:
Anbringen der Überwachungsvorrichtung (10) an einem saugfähigen Artikel (14), der ein Leiterbild umfasst,
Kontaktieren der mindestens drei Anschlüsse (11a, 11b, 11c) der Überwachungsvorrichtung (10) mit dem Leiterbild und
Überprüfen einer ordnungsgemäßen Anbringung der Überwachungsvorrichtung (10) an dem saugfähigen Artikel (14) mit Hilfe der Überwachungsvorrichtung (10).

12. Verfahren nach Anspruch 11, wobei das Verfahren die folgenden Schritte umfasst:
Wiederholen oder Korrigieren der Anbringung der Überwachungsvorrichtung (10) an dem saugfähigen Artikel (14) im Falle einer nicht ordnungsgemäßen Anbringung, bis die Überwachungsvorrichtung (10) ordnungsgemäß an dem saugfähigen Artikel (14) befestigt ist, und/oder
Identifizieren des Typs des saugfähigen Artikels (14) im Falle einer ordnungsgemäßen Anbringung mit Hilfe der Überwachungseinrichtung (10).

13. Verfahren nach Anspruch 12,
wobei das Verfahren den Schritt des Bestimmens des Nässe- und/oder Sättigungszustands des saugfähigen Artikels (14) mit Hilfe der Überwachungsvorrichtung (10) umfasst, insbesondere anhand des Typs des saugfähigen Artikels (14) und/oder insbesondere erst nach Überprüfung der ordnungsgemäßen Anbringung der Überwachungsvorrichtung an dem saugfähigen Artikel.

14. Saugfähiger Artikel (14), umfassend:
ein Leiterbild (21) und eine Überwachungsvorrichtung (10, 20) gemäß der Definition in Anspruch 1,
wobei das Leiterbild (21) Leiterbahnen umfasst,
wobei die Leiterbahnen derart ausgebildet sind, dass die Leiterbahnen mit mindestens drei Anschlüssen (11a, 11b, 11c) der Überwachungsvorrichtung (10, 20) nach einem vorgegebenen Muster miteinander verbinden, wenn das Leiterbild (21) durch die mindestens drei Anschlüsse (11a, 11b, 11c) der Überwachungsvorrichtung (10, 20) kontaktiert wird.

15. Saugfähiger Artikel (14) nach Anspruch 14, wobei das Leiterbild (21) eine Tinte umfasst, vorzugsweise eine Tinte auf Kohlenstoffbasis und/oder eine leitfähige Tinte auf Polymerbasis.

16. Saugfähiger Artikel (14) nach Anspruch 15, wobei die Tinte mindestens eines von Graphen, Graphit, Nano-Kohlenstoffröhren, Polyacetylen, Polypyrrol, Polyanilin und Copolymeren davon, Polypyrrolen (PPY), Polyanilinen (PANI), Polythiophenen (PT), Poly(p-phenylensulfid) (PPS), Poly(p-phenylen) (PPP), Polyacetylenen (PAC), Poly(p-phenylenvinylen) (PPV), Poly(3,4-ethylendioxythiophen) (PEDOT), Poly(3,4-ethylendioxythiophen)-Polystyrolsulfonat (PEDOT:PSS) oder eine beliebige Kombination davon umfasst.

## Revendications

1. Dispositif de surveillance (10) pouvant être fixé à un article absorbant (14) comprenant un motif conducteur, le dispositif de surveillance (10) comprenant :
au moins trois bornes (11a, 11b, 11c) pouvant être mises en contact avec le motif conducteur, dans lequel les bornes (11a, 11b, 11c) sont disposées sur le dispositif de surveillance (10) selon un motif de borne prédéfini, et dans lequel le motif de borne prédéfini comprend ou est une matrice ; et
une unité de traitement (12) connectée aux au moins trois bornes (11a, 11b, 11c),
dans lequel, dans le cas où le dispositif de surveillance (10) est fixé à l'article absorbant (14) et les au moins trois bornes (11a, 11b, 11c) entrent en contact avec le motif conducteur au moins partiellement, sur la base du contact entre au moins une partie des au moins trois bornes (11a, 11b, 11c) et le motif conducteur, l'unité de traitement (12) est configurée pour acquérir des informations permettant de vérifier une fixation correcte du dispositif de surveillance (10) à l'article absorbant (14) et/ou permettant d'identifier un type de l'article absorbant (14) et/ou permettant de déterminer un état d'humidité et/ou de saturation de l'article absorbant (14), dans lequel les informations acquises comprennent au moins une valeur de mesure d'au moins l'une parmi une résistance, une capacité, une inductance, une impédance ou leur combinaison quelconque

2. Dispositif de surveillance (10) selon la revendication 1, dans lequel le dispositif de surveillance (10) comprend au moins quatre bornes, de préférence six bornes, plus préférablement huit bornes, encore plus préférablement quatorze bornes.

3. Dispositif de surveillance (10) selon la revendication 1 ou 2, dans lequel la distance entre les bornes les unes par rapport aux autres est prédéfinie.

4. Dispositif de surveillance (10) selon la revendication 3, dans lequel la matrice comprend deux lignes et/ou deux colonnes.

5. Dispositif de surveillance (10) selon l'une quelconque des revendications 1 à 4,
dans lequel, pour vérifier la fixation correcte du dispositif de surveillance (10) à l'article absorbant (14), les informations acquises comprennent le résultat d'une comparaison entre l'au moins une valeur de mesure et au moins une valeur de référence de vérification respective, de préférence au moins une valeur seuil de vérification respective.

6. Dispositif de surveillance (10) selon l'une quelconque des revendications 1 à 5,
dans lequel, pour identifier le type de l'article absorbant (14), les informations acquises comprennent le résultat d'une comparaison entre l'au moins une valeur de mesure et au moins une valeur de référence d'identification respective, de préférence au moins une valeur seuil d'identification respective, et/ou le résultat d'une interprétation de type de l'au moins une valeur de mesure.

7. Dispositif de surveillance (10) selon l'une quelconque des revendications 1 à 6,
dans lequel, pour déterminer l'état d'humidité et/ou de saturation de l'article absorbant (14), les informations acquises comprennent le résultat d'une comparaison entre l'au moins une valeur de mesure et au moins une valeur de référence de détermination respective, de préférence au moins une valeur seuil de détermination respective, et/ou le résultat d'une interprétation d'état de l'au moins une valeur de mesure.

8. Dispositif de surveillance (10) selon l'une quelconque des revendications 1 à 7,
dans lequel, pour vérifier la fixation correcte du dispositif de surveillance (10) à l'article absorbant (14), l'unité de traitement (12) est configurée pour sélectionner au moins deux, de préférence au moins deux paires, des bornes relativement espacées ou ayant la plus grande distance l'une par rapport à l'autre pour l'acquisition des informations.

9. Dispositif de surveillance (10) selon l'une quelconque des revendications 1 à 7,
dans lequel, pour vérifier la fixation correcte du dispositif de surveillance (10) à l'article absorbant (14) et/ou pour identifier le type de l'article absorbant (14), l'unité de traitement (12) est configurée pour sélectionner au moins deux, de préférence au moins deux paires, des bornes pour l'acquisition des informations de manière à ce que les bornes sélectionnées soient sans ambiguïté court-circuitées ou laissées ouvertes en entrant en contact avec le motif conducteur.

10. Dispositif de surveillance (10) selon l'une quelconque des revendications 1 à 9,
dans lequel le dispositif de surveillance (10) comprend une unité de rétroaction (13),
dans lequel l'unité de rétroaction (13) est configurée pour notifier, par voie acoustique et/ou optique et/ou haptique, un état concernant au moins l'un parmi la fixation correcte du dispositif de surveillance (10) à l'article absorbant (14), le type de l'article absorbant (14), l'humidité et/ou la saturation de l'article absorbant (14), ou leur combinaison quelconque.

11. Procédé d'utilisation d'un dispositif de surveillance (10) selon l'une quelconque des revendications 1 à 10, le procédé comprenant les étapes consistant à :
fixer le dispositif de surveillance (10) à un article absorbant (14) comprenant un motif conducteur,
mettre en contact les au moins trois bornes (11a, 11b, 11c) du dispositif de surveillance (10) avec le motif conducteur, et
vérifier une fixation correcte du dispositif de surveillance (10) à l'article absorbant (14) à l'aide du dispositif de surveillance (10).

12. Procédé selon la revendication 11, dans lequel le procédé comprend les étapes consistant à :
répéter ou corriger la fixation du dispositif de surveillance (10) à l'article absorbant (14) en cas de mauvaise fixation jusqu'à ce que le dispositif de surveillance (10) soit correctement fixé à l'article absorbant (14), et/ou
identifier le type de l'article absorbant (14) en cas de fixation correcte à l'aide du dispositif de surveillance (10).

13. Procédé selon la revendication 12, dans lequel le procédé comprend l'étape consistant à déterminer l'état d'humidité et/ou de saturation de l'article absorbant (14) à l'aide du dispositif de surveillance (10), en particulier sur la base du type de l'article absorbant (14) et/ou en particulier seulement après avoir vérifié la fixation correcte du dispositif de surveillance à l'article absorbant.

14. Article absorbant (14) comprenant :
un motif conducteur (21) et un dispositif de surveillance (10,20) tel que défini dans la revendication 1
dans lequel le motif conducteur (21) comprend des pistes conductrices,
dans lequel les pistes conductrices sont formées de manière à ce que les pistes conductrices interconnectent au moins trois bornes (11a, 11b, 11c) dudit dispositif de surveillance (10, 20) selon un motif prédéfini si le motif conducteur (21) est contacté par les au moins trois bornes (11a, 11b, 11c) du dispositif de surveillance (10, 20).

15. Article absorbant (14) selon la revendication 14, dans lequel le motif conducteur (21) comprend une encre, de préférence une encre à base de carbone et/ou une encre à base de polymère conducteur.

16. Article absorbant (14) selon la revendication 15, dans lequel l'encre comprend au moins l'un parmi graphène, graphite, nanotubes de carbone, polyacétylène, polypyrrole, polyaniline et copolymères de ceux-ci, poly(pyrrole)s (PPY), polyanilines (PANI), poly(thiophène)s (PT), poly(sulfure de p-phénylène) (PPS), poly(p-phénylène) (PPP), poly(acétylène)s (PAC), poly(p-phénylène vinylène) (PPV), poly(3,4-éthylènedioxythiophène) (PEDOT), poly(3,4-éthylènedioxythiophène) polystyrène sulfonate (PEDOT :PSS), ou leur combinaison quelconque.
